# EUROPEAN PATENT APPLICATION

(11) **EP 0 898 966 A1**
(43) Date of publication of application: **03.03.1999**
(21) Application number: 98306577.2
(22) Date of filing: 18.08.1998
(51) Int. Cl.: A61K 31/445, A61K 31/38

(54) **Benzo(b)thiophene derivatives for inhibiting temporomandibular disorders**

(30) Priority: 21.08.1997 US 56200 P
(71) Applicant: ELI LILLY AND COMPANY, Indianapolis, Indiana 46285 (US)
(72) Inventor: Grese, Timothy Alan, Indianapolis, Indiana 46256 (US)
(74) Representative: Denholm, Anna Marie

(57) **Abstract**

The preparation of a pharmaceutical formulation for inhibiting temporomandibular disorder in a human using 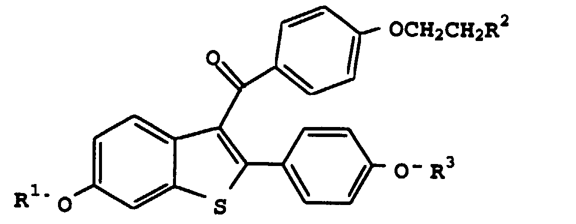 wherein R¹ and R³ are, independently, -H, -CH₃, -CO(C₁-C₆ alkyl), or -COAr, where Ar is optionally substituted phenyl;
R³ is selected from the group consisting of pyrrolidine, piperidine, and hexamethyleneimino; or
a pharmaceutically acceptable salt or solvate thereof.

## Description

The current invention deals with the areas of medicinal chemistry, pharmacology and clinical medicine related to the stet temporomandibular disorder (TMD).

Temporomandibular disorder (TMD), also known as TMJ, is a fairly common disease which effects as many as 15% of adult age people in North American continent. The most common deleterious symptoms of this disease are pain which can be mild to excruciating, loss or reduced use of the jaw effecting eating and speaking, and psychological manifestations such as depression caused by the constant discomfort.

Although the etiology of the disease is not well understood, the epidemiology is more definitive. The disease is several fold more prevalent in females, and particularly in women in their child bearing years. The disease usually appears at puberty and tends to abate at menopause. In addition, clinical studies of women taking estrogens either as hormone replacement therapy for menopausal subjects or as birth control therapy for premenopausal subjects, demonstrate the potential causal link between estrogen and TMD (see: "Use of Exogenous Hormones and Risk of Temporomadibular Disorder Pain", LeResche, L., *et al., Pain, 69,* pp. 153-160 (1997) and references cited therein, which are incorporated by reference herein).

The symptoms of TMD originate in the tissues associated with the mandibular joint. Although no direct relation has been established, it would seem reasonable that there may be a connection between the effect of estrogen on the laxity of joints in the pelvic region (associated with easing the tension of these joint in childbirth) and unintended effects on other joints such as the mandible. Such a loosening of the mandibular joint may be part of the mechanism of TMD.

A consequence of the potential association of TMD with the reproductive related agonist properties of estrogen is that an antagonist of these reproductive functions may also antagonize the effect of estrogen on the mandibular joint. Although many compounds are known to block the reproductive effects of estrogen, many of these compounds have detrimental side-effects such as osteoporosis, hyperlipidemia, and the like.

Currently, TMD is a very difficult disease to successfully treat. Therapies range from the use of opiates for pain to prothestic devices to position the jaw. Yet, most times, the results are less than satisfactory.

The current invention provides methods for inhibiting temporomandibular disorder in a human, which comprises the administration to a human in need thereof an effective amount of a compound of formula I 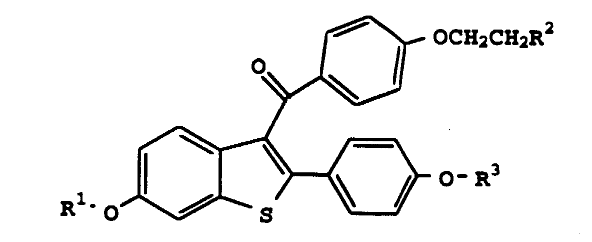 wherein R¹ and R³ are, independently, -H, -CH₃, -CO(C₁-C₆ alkyl), or -COAr, where Ar is optionally substituted phenyl;
R³ is selected from the group consisting of pyrrolidine, piperidine, and hexamethyleneimino; or
a pharmaceutically acceptable salt or solvate, thereof.

The current invention is related to the discovery that a select group of 2-aryl benzo[b]thiophenes (the compounds of formula I) are useful for inhibiting temporomandibular disorder in a human.

General terms used in the description of compounds herein described bear their usual meanings. For example, "C₁-C₆ alkyl" refers to straight or branched aliphatic chains of 1 to 6 carbon atoms including methyl, ethyl, propyl, iso-propyl, n-butyl, pentyl, hexyl and the like.

The term "substituted phenyl" refers to a phenyl group alone or having one or more substituents selected from the group consisting of C₁-C₄ alkyl, C₁-C₄ alkoxy, hydroxy, nitro, chloro, fluoro, or tri(chloro or fluoro)methyl. "OC₁-C₄ alkyl" refers a C₁-C₄ alkyl group attached through an oxygen bridge such as , methoxy, ethoxy, n-propoxy, iso-propoxy, and the like.

The term "pharmaceutically acceptable salt" refers to either acid or base addition salts which are known to be non-toxic and are commonly used in the pharmaceutical literature. Commonly used acid addition salts are inorganic salts formed by the addition of sulfuric acid, nitric acid, hydrochloric acid, hydrobromic acid phosphoric acid, phosphorous acid and the like; or organic salts formed by the addition of acetic acid, formic acid, benzoic acid, citric acid, methanesulfonic acid and the like. Commonly used basic addition salts are the salts formed by alkali or alkaline earth hydroxides, ammonium hydroxide, alkyl or aromatic amines and the like. A preferred salt of this invention is the hydrochloride salt.

The term "solvate" refers to a molecular complex of a compound of formula I with one or more solvent molecules. Such solvent molecules would be those commonly used in the pharmaceutical literature, which are known to be innocuous to the recipient, e.g., water, ethanol, and the like.

The term inhibit is defined to include its generally accepted meaning which includes slowing, stopping, and ameliorating. As such, the current invention encompasses both prophylactic and treatment therapies.

The compounds of this invention are derivatives of centrally located carbon, i.e., the "-CO-" moiety in formula I, thus derivatives are methanones, e.g., a compound of A-CO-B, would be named [A] [B]methanone. Further the compounds of formula I are derivatives of benzo[b]thiophene which is named and numbered according to the Ring Index, The American Chemical Society, as follows:

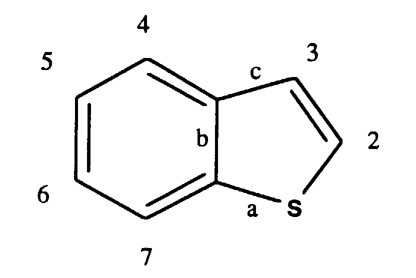

Thus, raloxifene hydrochloride, which is a preferred embodiment of this invention, is a compound of formula I, where R¹ and R³ are both hydrogen and R² is a piperidinyl ring, the hydrochloride salt thereof. Raloxifene hydrochloride is named [2-(4-hydroxyphenyl)-6-hydroxybenzo[b]thie-3-yl] [4-[2-(1-piperidenyl)ethoxy]phenyl]methanone hydrochloride.

All of the compounds used in the methods and formulations of the current invention can be made according to procedures, such as those detailed in US Pat. No. 4,133,814 and US Pat. No. 4,418,068, each of which is included by reference, herein. In general, the process starts with a benzo[b]thiophene having a 6-hydroxyl group and a 2-(4-hydroxylphenyl) group. The starting compound is protected, alkylated, and deprotected to form the compounds of formula I. The formula I compounds which are carboxylic esters may be prepared by methods described in US Pat. No. 5,393,763, which included by reference, herein.

The compounds of formula I are members of a group of compounds previously known as antiestrogens, but which have selective estrogenic agonist and antagonist pharmacologic activities. For example, formula I compounds act as estrogen agonists in treating pathologic sequelae caused by the cessation of menses in females (see: Draper et *al.,* "Effects of Raloxifene (Lyl39481 HCl) on Biochemical Markers of Bone and Lipid Metabolism in Healthy Postmenopausal Women", Hong Kong, Fourth Int'1. Symp. on Osteoporosis, March 29, 1993.; US Pat. Nos. 5,393,763, 5,464,845, and 5,391,557).

As used herein, the term "effective amount" means an amount of compound of the present invention which is capable of inhibiting TMD.

By "pharmaceutically acceptable formulation" it is meant that the carrier, diluent, solvent, excipients and salt must be compatible with the active ingredient (a compound of formula I) of the formulation, and not be deleterious to the recipient thereof.

Pharmaceutical formulations can be prepared by procedures known in the art. For example, the compounds of this invention can be formulated with common excipients, diluents, or carriers, and formed into tablets, capsules, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as agar agar, calcium carbonate, and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonire; and lubricants such as talc, calcium and magnesium stearate and solid polyethyl glycols. Final pharmaceutical forms may be pills, tablets, powders, lozenges, syrups, aerosols, saches, cachets, elixirs, suspensions, emulsions, ointments, suppositories, sterile injectable solutions, or sterile packaged powders, depending on the type of excipient used.

Additionally, the compounds of this invention are well suited to formulation as sustained release dosage forms. The formulations can also be so constituted that they release the active ingredient only or preferably in a particular part of the intestinal tract, possibly over a period of time. Such formulations would involve coatings, envelopes, or protective matrices which may be made from polymeric substances or waxes.

The particular dosage of a compound of formula I required to inhibit TMD according to this invention will depend upon the particular symptom and severity. Such considerations as a dosage, route of administration, and frequency of dosing are best decided by the attending physician. Generally, accepted and effective doses for oral or parenteral administration will be from 10mg to 800mg, and more typically between 20mg and 100mg. Such dosages will be administered to a patient in need of treatment from once to three times each day or as often as needed to effectively inhibit TMD.

The formulations which follow are given for purposes of illustration and are not intended to be limiting in any way. The total active ingredients in such formulations comprises from 0.1% to 99.9% by weight of the formulation. The term, "active ingredient" means a compound of formula I, preferably Raloxifene hydrochloride.

| Formulation 1: Gelatin Capsules | |
|---|---|
| Ingredient | Quantity (mg/capsule) |
| Active Ingredient | 50-600 |
| Starch NF | 0-500 |
| Starch flowable powder | 0-500 |
| Silicone fluid 350 centistrokes | 0-15 |

The ingredients are blended, passed through a No. 45 mesh U.S. sieve, and filled into hard gelatin capsules.

| Formulation 2: Tablets | |
|---|---|
| Ingredient | Quantity (mg/tablet) |
| Active Ingredient | 50-600 |
| Starch | 10-50 |
| Cellulose, microcrystalline | 10-20 |
| Polyvinylpyrrolidone | 5 |
| (as 10% solution in water) | |
| Sodium carboxymethyl cellulose | 5 |
| Magnesium stearate | 1 |
| Talc | 1-5 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules thus produced are dried at 50-60° C and passed through a No. 18 mesh U.S. sieve. The sodium carboxymethyl cellulose, magnesium stearate, and talc, previously passed through a No. 60 mesh U.S. sieve, are added to the above granules and thoroughly mixed. The resultant material is compressed in a tablet forming machine to yield the tablets.

| Formulation 3: Aerosol | |
|---|---|
| Ingredient | Weight % |
| Active Ingredient | 0.50 |
| Ethanol | 29.50 |
| Propellant 22 | 70.00 |
| (Chlorodifluoromethane) | |
| | |
| Total | 100.00 |

The active ingredient is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

| Formulation 4: Suspension | |
|---|---|
| Suspensions each containing 100 mg of a compound of formula I per 5 mL dose. | |

| Ingredient | Weight |
|---|---|
| Active Ingredient | 100 mg |
| Sodium carboxymethyl | |
| cellulose | 50 mg |
| Syrup | 1.25 mL |
| Benzoic acid solution (0.1M) | 0.10 mL |
| Flavor | q.v. |
| Color | q.v. |
| Purified water to total | Total 5 mL |

A compound of formula I is passed through a No. 45 mesh U.S. sieve and mixed with the sodium carboxymethyl cellulose and syrup to form a smooth paste. The benzoic acid solution, flavor, and color diluted in water are added and mixture stirred thoroughly. Additional water is added to bring the entire mixture to the required volume.

### Assay

One hundred women suffering from TMD are selected. Such patients are identified as suffering from TMD by diagnostic criteria which are found in the references, cited *supra.* Each patient is interviewed at the initial time point by a check list of the presence and severity of symptoms. This interview methods will be useful to evaluate the patient's progress throughout the duration of the clinical study. Because there are virtually no direct measurable criteria or end-points, i.e., most criteria are self-evaluated, a double-blind crossover study is probably the most objective method for assessing the efficacy of the therapy.

Thus, fifty patients are given a compound of formula I, e.g., Raloxifene hydrochloride at 60 mg per day via the oral route. Fifty patients are given a matched placebo. At the end of six months, all patients are interviewed and the results recorderd. The patients are crossed-over in their therapy and the study continues for an additional six months. The patients are again interviewed and the results recorded. The results are analyzed (see: references for specific methodologies). A positive result indicates that the patients when receiving a compound of formula I show less symptoms of TMD than those receiving placebo.

## Claims

1. The use of a compound of formula I 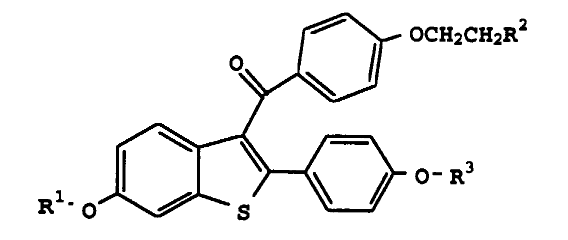 wherein R¹ and R³ are, independently, -H, -CH₃, -CO(C₁-C₆ alkyl), or -COAr, where Ar is optionally substituted phenyl;
R³ is selected from the group consisting of pyrrolidine, piperidine, and hexamethyleneimino; or
a pharmaceutically acceptable salt or solvate thereof, in the preparation of a pharmaceutical formulation for inhibiting temporomandibular disorder in a human..

2. A use according to Claim 1 wherein said compound is [2-(4-hydroxyphenyl) -6-hydroxybenzo[b]thien-3-yl][4-[2-(1-piperidinyl)ethoxy]phenyl]methanone hydrochloride.

3. A use according to Claim 2 wherein compound is administered in an amount of 60 mg/day via the oral route.
